# EUROPEAN PATENT APPLICATION

(11) **EP 4 799 569 A1**
(43) Date of publication of application: **02.09.2026**
(21) Application number: 23956746.4
(22) Date of filing: 24.10.2023
(51) Int. Cl.: A61B 5/16, A61B 5/00, A61B 5/01, A61B 5/0245, A61B 5/08

(54) **CARDIOPULMONARY ARREST PREDICTION DEVICE FOR MAMMAL**

(71) Applicant: Nakamura, Hiroshi, Iwakuni-shi, Yamaguchi 740-0724 (JP)
(72) Inventor: Nakamura, Hiroshi, Iwakuni-shi, Yamaguchi 740-0724 (JP)
(74) Representative: Germain Maureau
(86) International application number: PCT/JP2023/038315
(87) International publication number: WO 2025/088689

(57) **Abstract**

A cardiopulmonary arrest prediction device includes: a right sound detector and a left sound detector configured to detect sounds generated by heartbeat fluctuations at a right Yifeng site and a left Yifeng site and detect sounds generated by respiration fluctuations at the right Yifeng site and the left Yifeng site; a calculation section configured to calculate a stress index of a mammal based on the sounds generated by the heartbeat fluctuations and the sounds generated by the respiration fluctuations and detected by the right sound detector and the left sound detector; a prediction section configured to predict the timing of cardiopulmonary arrest in a mammal based on the stress index calculated by the calculation section; and a notification section configured to notify the timing of cardiopulmonary arrest predicted by the prediction section.

## Description

### TECHNICAL FIELD

The present disclosure relates to a cardiopulmonary arrest prediction device capable of predicting the timing of cardiopulmonary arrest in a mammal.

### BACKGROUND ART

For example, Patent Document 1 discloses a contact-type biological sound sensor fixed while inserted into an external auditory canal as an example of a sensor that acquires biological information. The contact-type biological sound sensor of Patent Document 1 includes: a biological sound sensor that acquires biological sound as the biological information; and an acoustic transmission portion made of a material having an acoustic impedance close to that of the skin.

Further, Patent Document 2 discloses a biological information acquisition device used while inserted into the external auditory canal and configured to extract a vascular sound waveform and a respiration sound waveform based on an acoustic signal acquired by a bone- and tissue-conducted sound sensor.

### CITATION LIST

### PATENT DOCUMENT

PATENT DOCUMENT 1: Japanese Unexamined Patent Publication No. 2021-74464
PATENT DOCUMENT 2: Japanese Patent No. 7287612

### SUMMARY OF THE INVENTION

### TECHNICAL PROBLEM

In circumstances in which a patient who is hospitalized due to, for example, a disease, injury, senility, or the like has no prospect of recovery and is approaching death, there is a demand to predict when cardiopulmonary arrest will occur in the patient.

It is possible to predict the timing of cardiopulmonary arrest by acquiring and analyzing various types of biological information of the patient. In this regard, as disclosed in Patent Document 1 and Patent Document 2, it is conceivable to acquire biological sounds, vascular sound waveforms, and the like by using a sensor inserted into an external auditory canal. However, when such biological information is used to predict the timing of cardiopulmonary arrest, an issue arises as to how to improve the accuracy of the information obtained. Furthermore, there is also a need to predict cardiopulmonary arrest in non-human mammals.

The present disclosure has been made in view of the above circumstances, and an object thereof is to enable accurate prediction of the timing of cardiopulmonary arrest in a mammal.

### SOLUTION TO THE PROBLEM

In order to achieve the above object, an aspect of the present disclosure can be premised on a cardiopulmonary arrest prediction device for predicting the timing of cardiopulmonary arrest in a mammal. The cardiopulmonary arrest prediction device includes: a right sound detector and a left sound detector configured to detect sounds generated by heartbeat fluctuations at a right Yifeng site and a left Yifeng site of a mammal and detect sounds generated by respiration fluctuations at the right Yifeng site and the left Yifeng site; a calculation section configured to calculate a stress index of a mammal based on the sounds generated by the heartbeat fluctuations and the sounds generated by the respiration fluctuations and detected by the right sound detector and the left sound detector; a prediction section configured to predict the timing of cardiopulmonary arrest in a mammal based on the stress index calculated by the calculation section; and a notification section configured to notify the timing of cardiopulmonary arrest predicted by the prediction section.

That is, since the right Yifeng site and the left Yifeng site of a mammal are close to arteries and airway, it is possible to accurately detect sounds related to heartbeat and sounds related to respiration. Furthermore, when sounds related to heartbeat change, such changes can be accurately detected as sounds generated by heartbeat fluctuations, and when sounds related to respiration change, such changes can be accurately detected as sounds generated by respiration fluctuations.

Thus, the heartbeat fluctuations and the respiration fluctuations can be accurately detected as the biological information of the mammal by the right sound detector and the left sound detector, so that the stress index calculated by the calculation section becomes a precise index indicating the current state of the mammal. The prediction section predicts the timing of cardiopulmonary arrest in the mammal based on the precise stress index; therefore, the accuracy of the predicted timing of cardiopulmonary arrest is improved. Since this timing of cardiopulmonary arrest is notified by the notification section, it becomes possible to know the timing of cardiopulmonary arrest with high accuracy.

The cardiopulmonary arrest prediction device for mammals may further include a right temperature detector and a left temperature detector configured to detect temperature fluctuations at the right Yifeng site and the left Yifeng site. In this case, the calculation section can calculate a stress index of a mammal based on the temperature fluctuations detected by the right temperature detector and the left temperature detector and on the sounds generated by the heartbeat fluctuations and the sounds generated by the respiration fluctuations and detected by the right sound detector and the left sound detector.

According to this configuration, at the right Yifeng site and the left Yifeng site, it is possible to accurately detect temperatures related to body temperature, and when the temperatures of the right Yifeng site and the left Yifeng site change, such changes can be accurately detected as temperature fluctuations. Since the timing of cardiopulmonary arrest in the mammal is predicted using the temperature fluctuations related to body temperature as well, the accuracy of the predicted timing of cardiopulmonary arrest is further improved.

The cardiopulmonary arrest prediction device for mammals may further include: a right sensor unit in which the right sound detector and the right temperature detector are integrated; and a left sensor unit in which the left sound detector and the left temperature detector are integrated. This makes it possible to easily detect the temperature fluctuations, the heartbeat fluctuations, and the respiration fluctuations simultaneously.

The calculation section can acquire a heart rate based on the sounds generated by the heartbeat fluctuations and estimate heart rate variability based on the acquired heart rate, acquire a respiration rate based on the sounds generated by the respiration fluctuations and estimate respiration rate variability based on the acquired respiration rate, and calculate a stress index of a mammal based on the estimated heart rate variability and the estimated respiration rate variability.

The calculation section can estimate body temperature variability based on the temperature fluctuations and calculate a stress index of a mammal based on the body temperature variability.

### ADVANTAGES OF THE INVENTION

As described above, the heartbeat fluctuations and the respiration fluctuations at the right Yifeng site and the left Yifeng site are detected, and the timing of cardiopulmonary arrest is predicted using a stress index calculated based on the detection results. Accordingly, the timing of cardiopulmonary arrest in the mammal can be accurately predicted.

### BRIEF DESCRIPTION OF THE DRAWINGS

[FIG. 1] FIG. 1 is a diagram showing the state of use of a cardiopulmonary arrest prediction device for a mammal according to an embodiment of the present invention.
[FIG. 2] FIG. 2 is a block diagram of the cardiopulmonary arrest prediction device for a mammal.
[FIG. 3] FIG. 3 is a diagram showing a correlation between a stress index and time before cardiopulmonary arrest (death).
[FIG. 4] FIG. 4 is a graph showing a relationship between a difference in left and right Yifeng temperatures of PCR-positive patients with mild symptoms and the presence or absence of the symptoms.
[FIG. 5] FIG. 5 is a graph showing forehead temperatures, left and right Yifeng temperatures, and axillary temperatures of PCR-positive patients with mild symptoms.
[FIG. 6] FIG. 6 is a photograph showing the state of VR rehabilitation treatment for phantom limb pain.

### DESCRIPTION OF EMBODIMENTS

Hereinafter, embodiments of the present invention will be described in detail with reference to the drawings. The following description of the preferred embodiments is merely exemplary in nature, and is not intended to limit the scope, application, or use of the present invention. The technical scope of the present invention is not limited to the embodiments below, and also includes other embodiments in which the configurations of the following embodiments are appropriately modified or substituted by a person skilled in the art.

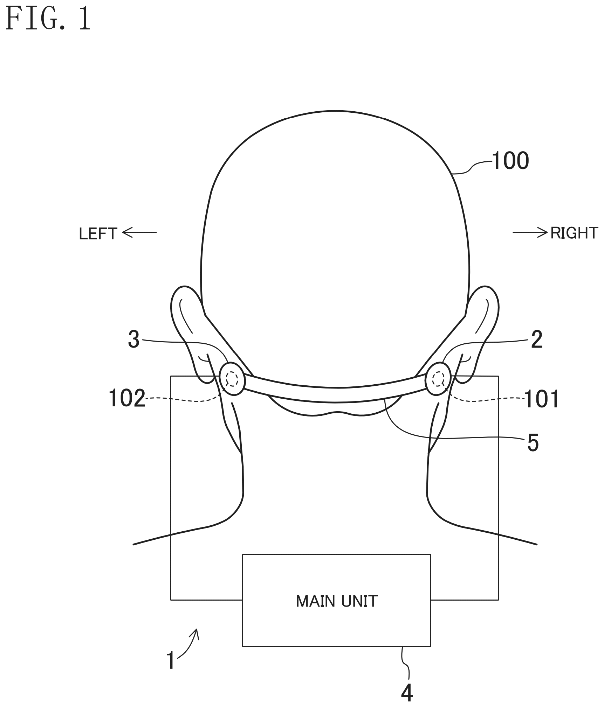

FIG. 1 is a diagram showing the state of use of a cardiopulmonary arrest prediction device for a mammal (hereinafter simply referred to as a "cardiopulmonary arrest prediction device 1") according to an embodiment of the present invention. In FIG. 1, reference numeral 100 denotes a person (prediction subject) for whom the timing of cardiopulmonary arrest is predicted by the cardiopulmonary arrest prediction device 1. In FIG. 1, since the prediction subject 100 is viewed from the rear side, the right side of the prediction subject 100 corresponds to the right side in FIG. 1, and the left side of the prediction subject 100 corresponds to the left side in FIG. 1. Immediately behind the earlobes and immediately behind the bases of the ears of the prediction subject 100, there are a right Yifeng 101 and a left Yifeng 102, which are acupuncture points in Eastern medicine (their approximate positions are indicated by broken lines). The Yifeng is a part which is depressed when the mouth is opened widely.

The cardiopulmonary arrest prediction device 1 according to the present invention can be applied not only to humans but also to non-human mammals. In non-human mammals as well, the right Yifeng 101 and the left Yifeng 102 are present similarly to humans.

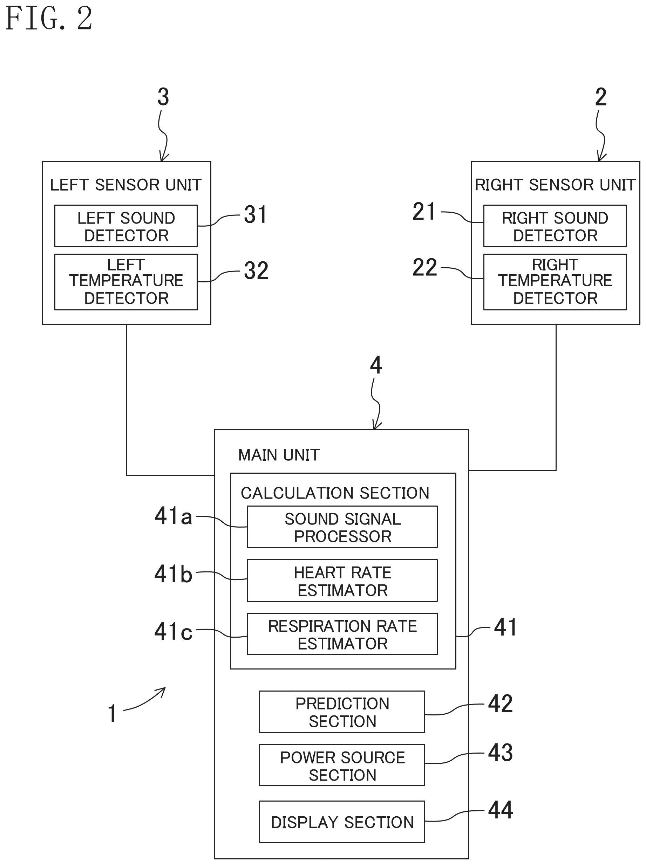

The cardiopulmonary arrest prediction device 1 includes a right sensor unit 2, a left sensor unit 3, and a main unit 4. As shown in FIG. 2, the right sensor unit 2 includes a right sound detector 21 and a right temperature detector 22, and is formed by integrating the right sound detector 21 and the right temperature detector 22. The right sensor unit 2 has a sound and temperature detection surface, and is disposed such that this detection surface is in contact with the surface (skin) of the right Yifeng 101 site. The left sensor unit 3 includes a left sound detector 31 and a left temperature detector 32, and is formed by integrating the left sound detector 31 and the left temperature detector 32. The left sensor unit 3 also has a sound and temperature detection surface, and is disposed such that this detection surface is in contact with the surface (skin) of the left Yifeng 102 site. The right sensor unit 2 and the left sensor unit 3 may be identical to each other.

In this embodiment, although not an essential element, a coupling portion 5 that couples the right sensor unit 2 and the left sensor unit 3 to each other is provided as shown in FIG. 1. The coupling portion 5 has, for example, a rod-like shape or a plate-like shape extending from the right sensor unit 2 to the left sensor unit 3, and is formed of an elastic member. The coupling portion 5 is configured to generate a biasing force in a direction that narrows the distance between the right sensor unit 2 and the left sensor unit 3. Thus, when the detection surface of the right sensor unit 2 is brought into contact with the surface of the right Yifeng 101 site and the detection surface of the left sensor unit 3 is brought into contact with the surface of the left Yifeng 102 site, the elasticity of the coupling portion 5 causes the detection surface of the right sensor unit 2 to be pressed against the surface of the right Yifeng 101 site and causes the detection surface of the left sensor unit 3 to be pressed against the surface of the left Yifeng 102 site. As a result, detachment of the right sensor unit 2 and the left sensor unit 3 from the prediction subject 100 is suppressed, so that sound and temperature at the right Yifeng 101 site and the left Yifeng 102 site can be continuously detected for a predetermined time or longer.

The right sound detector 21 and the left sound detector 31 are each configured as a sound detection sensor using, for example, an electret condenser microphone (ECM), and are members for detecting sounds generated by heartbeat fluctuations at the right Yifeng 101 site and the left Yifeng 102 site, respectively, and detecting sounds generated by respiration fluctuations at the right Yifeng 101 site and the left Yifeng 102 site, respectively. Since the right Yifeng 101 site and the left Yifeng 102 site are close to human arteries and airway, it is possible to accurately detect sounds related to heartbeat and sounds related to respiration. Furthermore, when sounds related to heartbeat change, such changes can be accurately detected as sounds generated by heartbeat fluctuations, and when sounds related to respiration change, such changes can be accurately detected as sounds generated by respiration fluctuations.

The sounds generated by the heartbeat fluctuations and the sounds generated by the respiration fluctuations, which are detected by the right sound detector 21 and the left sound detector 31, are converted into electric signals (acoustic signals) and transmitted to the main unit 4. That is, the right sound detector 21 and the left sound detector 31 are communicably connected to the main unit 4. The communication mode is not particularly limited, and may be wired communication using a wired communication module or wireless communication using a wireless communication module.

The right temperature detector 22 and the left temperature detector 32 are each configured as a temperature sensor capable of detecting temperatures, and are members for detecting temperature fluctuations at the right Yifeng 101 site and the left Yifeng 102 site, respectively. At the right Yifeng 101 and the left Yifeng 102, it is possible to accurately detect temperatures related to body temperature, and when the temperatures of the right Yifeng 101 site and the left Yifeng 102 site change, such changes can be accurately detected as temperature fluctuations. The temperature at the right Yifeng 101 site will be referred to as a right Yifeng temperature, and the temperature of the left Yifeng 102 site will be referred to as a left Yifeng temperature.

The temperature fluctuations detected by the right temperature detector 22 and the left temperature detector 32 are converted into electric signals and transmitted to the main unit 4.

As shown in FIG. 2, the main unit 4 includes a calculation section 41, a prediction section 42, a power source section 43, and a display section 44. The calculation section 41 and the prediction section 42 are configured by, for example, a microcomputer including a processor and a memory. The memory is configured to store computer-readable instructions (programs). For example, the memory may be a ROM storing various programs and a RAM having a plurality of work areas storing various programs to be executed by the processor. The memory may also be configured as a flash memory or the like. The processor is, for example, a CPU, an MPU, and/or a GPU. The CPU may include a plurality of CPU cores. The GPU may include a plurality of GPU cores. The main unit 4 may further include a storage section (not shown) configured by a storage such as an HDD, an SSD, and a flash memory. The storage section can store various types of data.

Although the calculation section 41 and the prediction section 42 are shown separately in FIG. 2, these components may be configured as one microcomputer. The power source section 43 supplies power to the calculation section 41, the prediction section 42, and the display section 44, and receives power input from, for example, a battery or a commercial power source. The power source section 43 can also supply power to the right sensor unit 2 and the left sensor unit 3 as necessary. The display section 44 is configured, for example, as a liquid crystal display, an organic EL display, or the like.

The calculation section 41 includes a sound signal processor 41a, a heart rate estimator 41b, and a respiration rate estimator 41c. The sound signal processor 41a receives the acoustic signals output from the right sound detector 21 and the left sound detector 31. When receiving the right-side acoustic signal output from the right sound detector 21, the sound signal processor 41a performs processing on the acoustic signal of a predetermined time of about 30 seconds, for example. Specifically, the sound signal processor 41a performs Fourier transform processing on the acoustic signal of the predetermined time to generate a right-side transformed signal. The acoustic signal of a predetermined time output from the left sound detector 31 is also subjected to Fourier transform processing to generate a left-side transformed signal.

The right-side and left-side transformed signals generated by the sound signal processor 41a are transmitted to the heart rate estimator 41b and the respiration rate estimator 41c. The heart rate estimator 41b includes a high-pass filter as a low-frequency component remover for removing low-frequency components, and inputs each of the right-side and left-side transformed signals to the high-pass filter to remove signals having a frequency of less than 0.8 Hz, thereby generating a right-side high-pass filtered signal and a left-side high-pass filtered signal. The heart rate estimator 41b determines a peak formed by a signal having an intensity equal to or greater than a predetermined intensity among the right-side high-pass filtered signals. The heart rate is acquired based on the peak determined from the right-side high-pass filtered signal. Similarly, the heart rate estimator 41b determines a peak formed by a signal having an intensity equal to or greater than a predetermined intensity among the left-side high-pass filtered signals. The heart rate is acquired based on the peak determined from the left-side high-pass filtered signal. In this manner, the calculation section 41 acquires the heart rate based on the sounds generated by the heartbeat fluctuations detected by the right sound detector 21 and the left sound detector 31, and estimates heart rate variability based on the acquired heart rate.

The respiration rate estimator 41c includes a low-pass filter as a high-frequency component remover for removing high-frequency components, and inputs each of the right-side and left-side transformed signals to the low-pass filter to remove signals having a frequency equal to or higher than 0.5 Hz, thereby generating a right-side low-pass filtered signal and a left-side low-pass filtered signal. The respiration rate estimator 41c determines a peak formed by a signal having an intensity equal to or greater than a predetermined intensity among the right-side low-pass filtered signals. The respiration rate is acquired based on the peak determined from the right-side low-pass filtered signal. Similarly, the respiration rate estimator 41c determines a peak formed by a signal having an intensity equal to or greater than a predetermined intensity among the left-side low-pass filtered signals. The respiration rate is acquired based on the peak determined from the left-side low-pass filtered signal. In this manner, the calculation section 41 acquires the respiration rate based on the sounds generated by the respiration fluctuations detected by the right sound detector 21 and the left sound detector 31, and estimates respiration rate variability based on the acquired respiration rate.

The calculation section 41 calculates a stress index of the mammal based on the temperature fluctuations detected by the right temperature detector 22 and the left temperature detector 32 and on the sounds generated by the heartbeat fluctuations and the sounds generated by the respiration fluctuations and detected by the right sound detector 21 and the left sound detector 31. The stress index (SI) is calculated based on the heart rate variability estimated by the heart rate estimator 41b and the respiration rate variability estimated by the respiration rate estimator 41c.

Specifically, the stress index is calculated using Expression 1 below. $\begin{matrix}Stress Index \left(SI\right) = Parasympathetic Nerve Activity / Sympathetic Nerve Activity \\ Respiration Rate Variability \left(σRR\right) / Heart Rate Variability \left(σHR\right)\end{matrix}$

In the above Expression 1, σ is a variance.

When an electrocardiogram is subjected to frequency analysis to obtain a power spectrum, the power spectrum is divided into two regions: LF (Low Frequency) and HF (High Frequency). The LF component increases when the sympathetic nerve or the parasympathetic nerve is activated, whereas the HF component increases when the parasympathetic nerve is activated. Thus, by using LF/HF as an index, it can be determined that the mammal is stressed when the numerical value is high and is relaxed when the numerical value is low.

It is also possible to evaluate the stress index by extracting the high-frequency fluctuation component (HF component) corresponding to the respiration rate variability and the low-frequency component (LF component) corresponding to a Mayer wave (blood pressure variability), and comparing the magnitudes of these components. That is, the calculation section 41 can calculate LF/HF as the stress index, which is a ratio between the high-frequency fluctuation component corresponding to the respiration rate variability and the low-frequency component corresponding to the Mayer wave (blood pressure variability).

For example, LF/HF can be evaluated using the sum (integral) of the intensities in the LF component region (from 0.05 Hz to 0.15 Hz) and the HF component region (from 0.15 Hz to 0.40 Hz) of the power spectrum.

In a relaxed state, that is, when the parasympathetic nerve is activated, the HF component reflecting the respiration rate variability and the LF component reflecting the blood pressure variability also appear. In a stressed state, that is, when the sympathetic nerve is activated, the LF component appears while the HF component decreases. Thus, in a relaxed state, the HF component becomes relatively large, and the value of LF/HF becomes small. Conversely, in a stressed state, the LF component becomes large relative to the HF component, and the value of LF/HF becomes large.

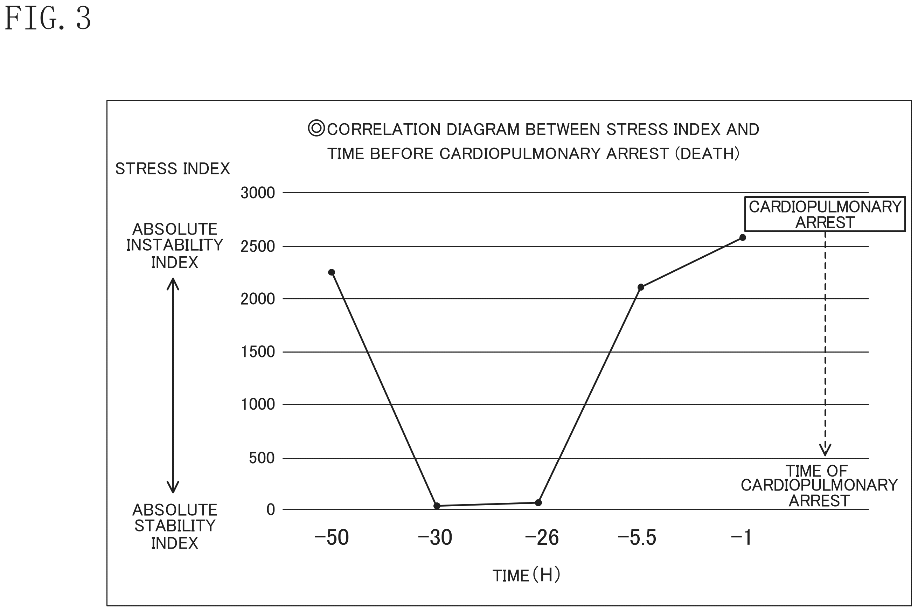

For example, FIG. 3 shows a correlation between the stress index and time before cardiopulmonary arrest (death). The vertical axis represents the stress index, and the horizontal axis represents the time of cardiopulmonary arrest. Absolute instability and absolute stability are signs of death observed about 48 hours prior to cardiopulmonary arrest (death). Observation using the cardiopulmonary arrest prediction device 1 reveals the following pattern in terminal patients.

The following pattern is shown as the sign of death observed approximately 48 hours prior to death.

Stable stress index → absolute instability of stress index (upward movement in the graph in the figure) → absolute stability (downward movement in the graph in the figure) → absolute instability (upward movement in the graph in the figure) → progression of absolute instability (further upward movement in the graph) → cardiopulmonary arrest (death)

That is, when the prediction section 42 acquires the stress index calculated by the calculation section 41 and analyzes variability, the time of cardiopulmonary arrest (death) can be predicted using the above-described pattern.

The calculation section 41 can also estimate body temperature variability based on the temperature fluctuations detected by the right temperature detector 22 and the left temperature detector 32. The right Yifeng 101 site and the left Yifeng 102 site are portions where the body temperature variability in mammals is likely to appear. By detecting temperatures at the right Yifeng 101 site and the left Yifeng 102 site, the body temperature variability in mammals can be accurately estimated. In this case, the calculation section 41 calculates the stress index based on the heart rate variability estimated by the heart rate estimator 41b, the respiration rate variability estimated by the respiration rate estimator 41c, and the body temperature variability.

In a case where the body temperature variability is included, the stress index is calculated using Expression 2 below. $\begin{matrix}Stress Index \left(SI\right) = \\ Respiration Rate Variability \left(σRR\right) \times Heart Rate Variability \left(σHR\right) \times Body \\ Temperature Variability \left(σBT\right)\end{matrix}$

In the above Expression 2, σ is a variance.

The prediction section 42 predicts the timing of cardiopulmonary arrest in the mammal based on the stress index calculated by the calculation section 41. For example, the larger the stress index calculated by the calculation section 41, the closer the timing of cardiopulmonary arrest is predicted to be. Conversely, the smaller the stress index calculated by the calculation section 41, the farther the timing of cardiopulmonary arrest is predicted to be. The timing of cardiopulmonary arrest can be predicted as a time period, such as the number of hours or minutes from the current time, or as the date and time of cardiopulmonary arrest.

The display section 44 displays the timing of cardiopulmonary arrest predicted by the prediction section 42. For example, the display section 44 can display the number of hours or minutes from the current time until the occurrence of cardiopulmonary arrest, or the date and time of cardiopulmonary arrest. In this manner, the timing of cardiopulmonary arrest of the mammal can be recognized by a user (such as a medical professional) of the cardiopulmonary arrest prediction device 1. The display section 44 is an example of a notification section that notifies the timing of cardiopulmonary arrest predicted by the prediction section 42.

Further, the cardiopulmonary arrest prediction device 1 may include a speaker (not shown) or the like. In this case, the timing of cardiopulmonary arrest predicted by the prediction section 42 can be made known to the user of the cardiopulmonary arrest prediction device 1 by voice. The speaker is another example of the notification section that notifies the timing of cardiopulmonary arrest predicted by the prediction section 42.

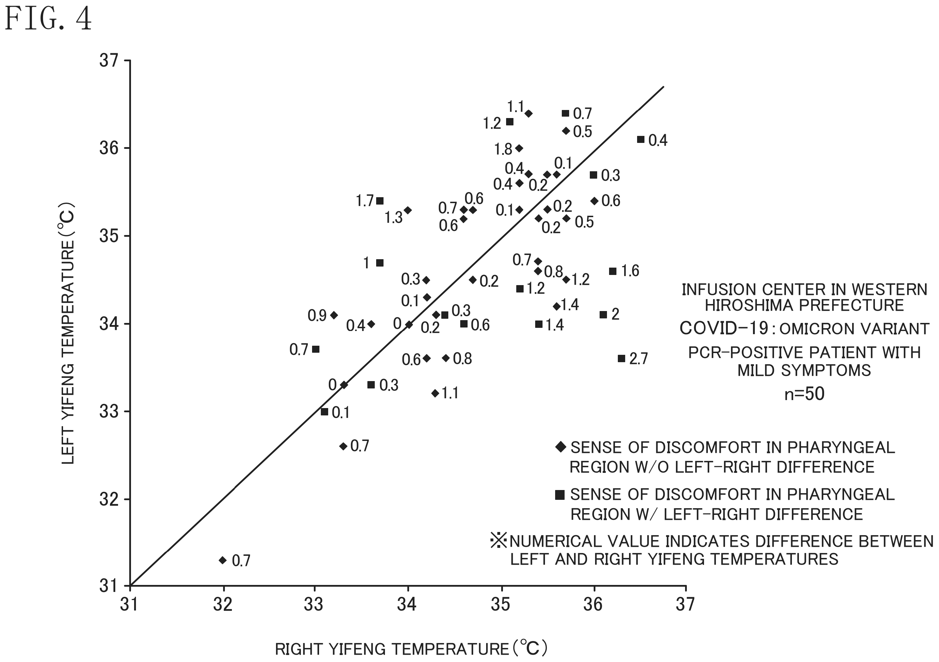

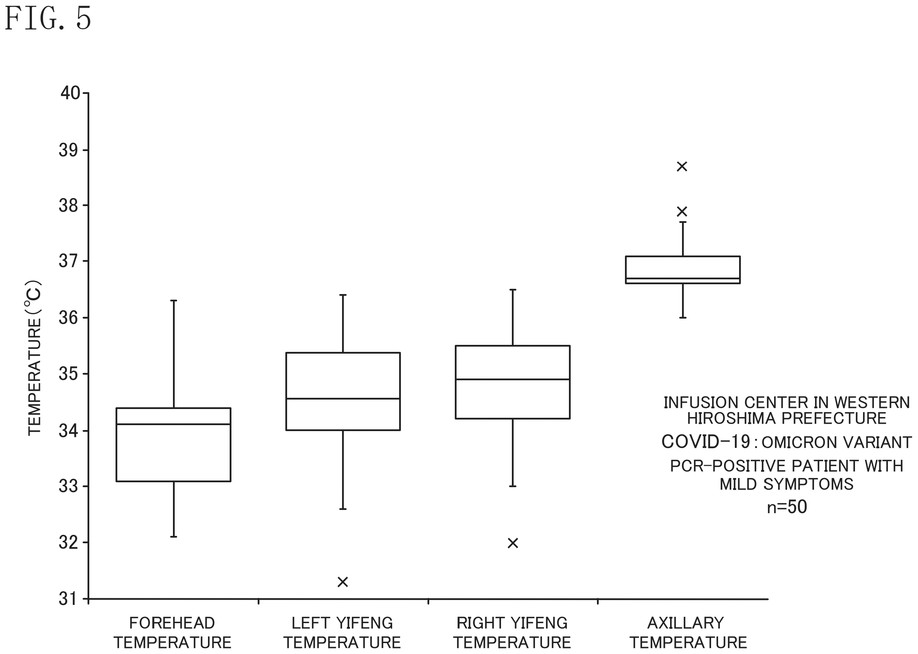

Here, an example will be described in which the temperatures at the right Yifeng 101 site and the left Yifeng 102 site precisely indicate the condition of the prediction subject 100. FIG. 4 is a graph showing a relationship between a difference between the left and right Yifeng temperatures and the presence or absence of a symptom (a sense of discomfort in the pharyngeal region) for 50 PCR-positive patients with mild symptoms who were determined to be infected with the Omicron variant (a type of COVID-19) by a PCR test. The measurement location was an infusion center in western Hiroshima Prefecture. The vertical axis of the graph represents the left Yifeng temperature (°C), and the horizontal axis represents the right Yifeng temperature (°C). The numerical values in the graph is the difference (°C) between the left Yifeng temperature and the right Yifeng temperature. FIG. 5 is a graph showing the forehead temperature, the left and right Yifeng temperatures, and the axillary temperature of the PCR-positive patients with mild symptoms.

When a doctor examined the 50 PCR-positive patients with mild symptoms in the graph of FIG. 4, it was found that, even among patients determined to be infected by the PCR test, if the difference between the left Yifeng temperature and the right Yifeng temperature was small, symptoms due to infection with the Omicron variant were mild regardless of whether there was a left-right difference in the sense of discomfort in the pharyngeal region, and in most cases, the patients were ultimately diagnosed as requiring only follow-up. On the other hand, in most patients with a difference of 1°C or more between the left Yifeng temperature and the right Yifeng temperature and a left-right difference in the sense of discomfort in the pharyngeal region, the symptoms due to infection with the Omicron variant were severe, and it was determined that treatment such as medication was immediately necessary.

As described above, the left Yifeng temperature and the right Yifeng temperature are biological information that not only indicates the temperature related to the body temperature of a mammal but also accurately indicates the condition of the mammal. Thus, by calculating the stress index using fluctuations in the left Yifeng temperature and the right Yifeng temperature, the calculation accuracy of the stress index of the mammal is improved. Further, infectious disease monitoring is also possible by using the left Yifeng temperature and the right Yifeng temperature. In addition, remote medical monitoring of patients with triglyceride deposit cardiomyovasculopathy (TGCV) is also possible by using the left Yifeng temperature and the right Yifeng temperature.

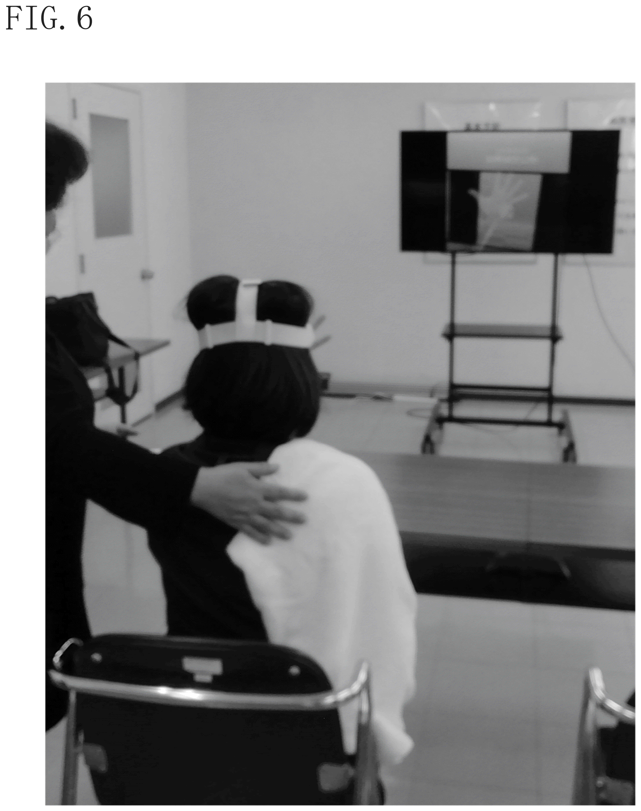

FIG. 6 is a photograph showing a state of VR rehabilitation treatment for phantom limb pain. VR rehabilitation is rehabilitation performed by moving the hands, feet, or the like in accordance with images displayed on virtual-reality goggles while the patient is wearing the goggles. Not only the rehabilitation for phantom limb pain but also rehabilitation treatment for sensory hypersensitivity can be performed.

By attaching the right sensor unit 2 and the left sensor unit 3 to the patient during the rehabilitation, the patient's right Yifeng temperature and left Yifeng temperature can be measured simultaneously. The following shows measurement results of blood pressure, pulse, oxygen saturation, right Yifeng temperature, and left Yifeng temperature of Patients 1 to 3 before and after they were subjected to higher brain function rehabilitation.

### Patient 1

| | Higher Brain Function Training (Before) | Higher Brain Function Training (After) |
|---|---|---|
| Blood Pressure (Systolic/Diastolic) | 197/73 | 164/80 |
| Pulse | 70 | 73 |
| Oxygen Saturation | 99% | 100% |
| Right Yifeng Temperature | 34.6°C | 36.0°C |
| Left Yifeng Temperature | 34.4°C | 36.1°C |

### Patient 2

| | Higher Brain Function Training (Before) | Higher Brain Function Training (After) |
|---|---|---|
| Blood Pressure (Systolic/Diastolic) | 195/83 | 182/76 |
| Pulse | 75 | 69 |
| Oxygen Saturation | 97% | 99% |
| Right Yifeng Temperature | 35.4°C | 36.3°C |
| Left Yifeng Temperature | 34.5°C | 36.0°C |

### Patient 3

| | Higher Brain Function Training (Before) | Higher Brain Function Training (After) |
|---|---|---|
| Blood Pressure (Systolic/Diastolic) | 165/71 | 156/72 |
| Pulse | 63 | 64 |
| Oxygen Saturation | 98% | 96% |
| Right Yifeng Temperature | 35.3°C | 35.4°C |
| Left Yifeng Temperature | 35.3°C | 35.6°C |

As described above, the right Yifeng temperature and the left Yifeng temperature after the rehabilitation are higher than those before the rehabilitation. This is because using the brain improves blood flow in the brain, which in turn increases the right Yifeng temperature and the left Yifeng temperature. From this fact as well, it can be understood that the left Yifeng temperature and the right Yifeng temperature are biological information capable of accurately indicating the condition of a mammal.

The embodiments above are merely examples in all respects and should not be construed as limiting. Further, all modifications and changes that fall within the scope of equivalents of the claims are within the scope of the present invention.

### INDUSTRIAL APPLICABILITY

As described above, the cardiopulmonary arrest prediction device for mammals according to the present disclosure can be used, for example, when predicting the timing of cardiopulmonary arrest in humans.

### DESCRIPTION OF REFERENCE CHARACTERS

- 1: Cardiopulmonary Arrest Prediction Device For Mammal
- 2: Right Sensor Unit
- 3: Left Sensor Unit
- 21: Right Sound Detector
- 22: Right Temperature Detector
- 31: Left Sound Detector
- 32: Left Temperature Detector
- 41: Calculation Section
- 42: Prediction Section
- 44: Display Section (Notification Section)

## Claims

1. A cardiopulmonary arrest prediction device for predicting timing of cardiopulmonary arrest in a mammal, the device comprising:
a right sound detector and a left sound detector configured to detect sounds generated by heartbeat fluctuations at a right Yifeng site and a left Yifeng site of a mammal and detect sounds generated by respiration fluctuations at the right Yifeng site and the left Yifeng site;
a calculation section configured to calculate a stress index of a mammal based on the sounds generated by the heartbeat fluctuations and the sounds generated by the respiration fluctuations and detected by the right sound detector and the left sound detector;
a prediction section configured to predict the timing of cardiopulmonary arrest in a mammal based on the stress index calculated by the calculation section; and
a notification section configured to notify the timing of cardiopulmonary arrest predicted by the prediction section.

2. The device of claim 1, further comprising:
a right temperature detector and a left temperature detector configured to detect temperature fluctuations at the right Yifeng site and the left Yifeng site, wherein
the calculation section calculates a stress index of a mammal based on the temperature fluctuations detected by the right temperature detector and the left temperature detector and on the sounds generated by the heartbeat fluctuations and the sounds generated by the respiration fluctuations and detected by the right sound detector and the left sound detector.

3. The device of claim 2, further comprising:
a right sensor unit in which the right sound detector and the right temperature detector are integrated; and
a left sensor unit in which the left sound detector and the left temperature detector are integrated.

4. The device of claim 1, wherein
the calculation section acquires a heart rate based on the sounds generated by the heartbeat fluctuations and estimates heart rate variability based on the acquired heart rate, acquires a respiration rate based on the sounds generated by the respiration fluctuations and estimates respiration rate variability based on the acquired respiration rate, and calculates a stress index of a mammal based on the estimated heart rate variability and the estimated respiration rate variability.

5. The device of claim 2, wherein
the calculation section estimates body temperature variability based on the temperature fluctuations and calculates a stress index of a mammal based on the body temperature variability.

## Amended claims

### Amended claims under Art. 19.1 PCT

1. A cardiopulmonary arrest prediction device for predicting timing of cardiopulmonary arrest in a mammal, the device comprising:
a right sound detector and a left sound detector configured to detect sounds generated by heartbeat fluctuations at a right Yifeng site and a left Yifeng site of a mammal and detect sounds generated by respiration fluctuations at the right Yifeng site and the left Yifeng site;
a calculation section configured to calculate a stress index of a mammal based on the sounds generated by the heartbeat fluctuations and the sounds generated by the respiration fluctuations and detected by the right sound detector and the left sound detector;
a prediction section configured to predict the timing of cardiopulmonary arrest in a mammal based on the stress index calculated by the calculation section;
a notification section configured to notify the timing of cardiopulmonary arrest predicted by the prediction section; and
a right temperature detector and a left temperature detector configured to detect temperature fluctuations at the right Yifeng site and the left Yifeng site, wherein
the calculation section calculates a stress index of a mammal based on the temperature fluctuations detected by the right temperature detector and the left temperature detector and on the sounds generated by the heartbeat fluctuations and the sounds generated by the respiration fluctuations and detected by the right sound detector and the left sound detector.

2. The device of claim 1, further comprising:
a right sensor unit in which the right sound detector and the right temperature detector are integrated; and
a left sensor unit in which the left sound detector and the left temperature detector are integrated.

3. The device of claim 1, wherein
the calculation section acquires a heart rate based on the sounds generated by the heartbeat fluctuations and estimates heart rate variability based on the acquired heart rate, acquires a respiration rate based on the sounds generated by the respiration fluctuations and estimates respiration rate variability based on the acquired respiration rate, and calculates a stress index of a mammal based on the estimated heart rate variability and the estimated respiration rate variability.

4. The device of claim 1, wherein
the calculation section estimates body temperature variability based on the temperature fluctuations and calculates a stress index of a mammal based on the body temperature variability.
